# EUROPÄISCHE PATENTSCHRIFT

(11) **EP 1 141 019 B1**
(45) Veröffentlichungstag und Bekanntmachung des Hinweises auf die Patenterteilung: **21.04.2004**
(21) Anmeldenummer: 99969227.0
(22) Anmeldetag: 22.12.1999
(51) Int. Cl.: C07K 14/705, A61K 38/17, G01N 33/68

(54) **FRAGMENT DES GLYCOPROTEINS CD55/DAF ZUR GEWINNUNG HOCHWIRKSAMER TUMORARZNEIEN SOWIE VERFAHREN ZU DESSEN VERWENDUNG**
SUBSTANCE FOR PRODUCING HIGHLY EFFECTIVE ANTI-TUMOUR MEDICAMENTS AND CORRESPONDING METHOD
SUBSTANCE POUR L'OBTENTION DE MEDICAMENTS ANTITUMORAUX HAUTEMENT EFFICACES, ET PROCEDE ASSOCIE

(30) Priorität: 22.12.1998 DE 19859248; 05.03.1999 DE 19909771
(43) Veröffentlichungstag der Anmeldung: 10.10.2001
(73) Patentinhaber: Vollmers, Heinz Peter, 97084 Würzburg (DE); Müller-Hermelink, Hans Konrad, 97082 Würzburg (DE)
(72) Erfinder: Vollmers, Heinz Peter, 97084 Würzburg (DE); Müller-Hermelink, Hans Konrad, 97082 Würzburg (DE)
(74) Vertreter: Weiss, Wolfgang, Dipl.-Chem. Dr.
(86) Internationale Anmeldenummer: PCT/EP1999/010329
(87) Internationale Veröffentlichungsnummer: WO 2000/037489

(56) Entgegenhaltungen:
- WO-A-99/43800
- US-A- 5 763 224
- BIOLOGICAL ABSTRACTS, vol. 1997, 1997 Philadelphia, PA, US; abstract no. 68043, L BJORGE ET AL.: "Complement-regulatory proteins in ovarian malignancies " XP002142921 & INTERNATIONAL JOURNAL OF CANCER, Bd. 70, Nr. 1, 1997, Seiten 14-25, NEW YORK, NY, US ISSN: 0020-7136
- F HENSEL ET AL.: "Characterization of glycosylphosphatidylinositol-linked molecule CD55/Decay-accelerating factor as the receptor for antibody SC-1-induced apoptosis" CANCER RESEARCH, Bd. 59, 15. Oktober 1999 (1999-10-15), Seiten 5299-5306, XP002142920 MD US

## Beschreibung

Die Erfindung betrifft eine Substanz sowie ein Verfahren zur Gewinnung von Antitumormitteln.

Beim Magenkarzinom handelt es sich um eine der weltweit häufigsten Krebsarten. Nach Lauren "The two histological main types of gastric carcinoma", Acta Path Microbiol Scand; 64:331-49, werden sie histologisch eingeteilt in diffuse Adenokarzinome und intestinale Adenokarzinome. Intestinale Magenkarzinome sind oft von chronischer Gastritis B begleitet und insbesondere von intestinalen Metaplasien, die als Vorläufer dysplastischer Veränderungen und von Magenkarzinomen betrachtet werden. Unterschiede zwischen diesen beiden Arten zeigen sich auch darin, daß Patienten mit Karzinomen des diffusen Typs oft der Blutgruppe A angehören, woraus auf den Einfluß genetischer Faktoren beim Krebsrisiko geschlossen werden kann, während Umweltfaktoren, z.B. eine Helicobacter pylori-Infektion, möglicherweise für die Entstehung von Karzinomen des intestinalen Typs von Bedeutung sind. Zwar ist eine abnehmende Häufigkeit der Magenadenokarzinome im Westen festzustellen, dafür treten sie aber nun vermehrt im Osten auf.

Die Therapie war bislang auf Gastrektomie und Lymphadenektomie beschränkt, aufgrund der auch dann noch schlechten Prognose besteht jedoch der Bedarf nach einer neuen begleitenden Therapie. Immunologische Studien haben gezeigt, daß auch in Fällen, in denen das Immunsystem maligne Zellen nicht wirksam bekämpfen kann. Zwar ist eine zelluläre und humorale Aktivität meßbar, aber nicht ausreichend, um die Tumorzellen zu zerstören. Ein wirkungsvoller Ansatz ist nun der, von der Immunantwort des Patienten stammende Antikörper zu isolieren, geeignet zu vermehren und therapheutisch einzusetzen. So wurden beispielsweise von Patienten mit Lungen-, Ösophagus- und Dickdarmkrebs stammende Antikörper isoliert und davon humane monoklonale Antikörper abgeleitet, die z.B. direkt Differentiation und das Wachstum der Tumorzellen beeinflussen, welche aber zumeist das Problem der Wechselwirkung mit anderen Tumoren oder gesunden Zellen haben.

Es ist bekannt, daß humane monoklonale SC-1-Antikörper Apoptose bei Magenkarzinomzellen auslösen können (Vollmers et al., Cancer 49 (1989), 2471-2476). Der Antikörper reagiert mit nahezu allen Adenokarzinomen vom diffusen Typ und etwa 20 % der Adenokarzinome vom intestinalen Typ (Vollmers et al., Cancer 76 (1995), 550-558; Vollmers et al., Cancer 79 (1997), 433-440). In klinischen Untersuchungen wurde gefunden, daß der Antikörper SC-1 in der Lage ist, eine tumorspezifische Regression und Apoptose bei primärem Magenkrebs ohne toxische Kreuzreaktivität gegenüber normalem Gewebe zu induzieren (Vollmers et al., Oncol. Rep. 5 (1998), 549-552).

Apoptose ist der programmierte Zelltod, Selbstmord von Zellen, durch Fragmentation der DNA, Zellschrumpfung und Dilatation des endoplasmatischen Reticulums, gefolgt von Zellfragmentation und der Bildung von Membranvesikeln, den sog. apoptotischen Körpern. Apoptose, die physiologische Form des Zelltods, garantiert eine schnelle und saubere Entfernung unnötiger Zellen, ohne Entzündungsvorgänge oder Gewebsverletzungen auszulösen wie im Falle der Nekrose. Unter pathologischen Bedingungen dient sie auch zum Entfernen maligner Zellen, wie etwa Krebsvorläuferzellen. Sie kann durch verschiedenste Stimuli ausgelöst werden, wie etwa durch zytotoxische T-Lymphozyten oder Zytokine, wie Tumornekrosefaktor, Glukokortikoide und Antikörper. Sie ist die häufigste Todesursache eukaryontischer Zellen und kommt vor in der Embryogenese, Metamorphose und Gewebsatrophie. Apoptotische Rezeptoren an der Zelloberfläche, wie jene der NGF/TNF-Familie werden prädominant auf Lymphozyten exprimiert, befinden sich aber auch auf verschiedenen anderen Zelltypen, weshalb sie sich nicht für eine Krebstherapie eignen. Insbesondere haben bei in-vivo-Tests Liganden und Antikörper für diese Rezeptoren zu Leberschäden geführt. Deshalb sind tumorspezifische Rezeptoren mit apoptotischer Funktion besonders wichtig.

Der zelluläre Rezeptor des monoklonalen Antikörpers SC-1 war bisher nicht bekannt. Im Rahmen der zur vorliegenden Erfindung führenden Untersuchungen konnte dieser zelluläre Rezeptor identifiziert werden. Diese Identifizierung gestaltete sich jedoch als schwierig. Einerseits reagiert der monoklonale Antikörper SC-1 bei der Westernblot-Analyse mit seinem Rezeptor nur unter ganz bestimmten Stringenzbedingungen. Andererseits findet man eine durch Denaturierungsartefakte hervorgerufene unspezifische Reaktion mit einer Reihe weiterer Proteine.

Bei dem zellulären Rezeptor des Antikörpers SC-1 handelt es sich um eine für Tumorzellen, insbesondere für Magenkarzinomzellen spezifische Isoform des Proteins CD55/DAF (Medof et al., J. Exp. Med. 160 (1984), 1558-1578; Caras et al., Nature 325 (1987), 545-549; Bjorge et al., Int. J. Cancer 70 (1997), 14-25), die in normalem Gewebe nicht auftritt. Die spezifischen Rezeptoreigenschaften dieser Isoform beruhen auf einer besonderen mitdem Proteinrückgrat über eine N-Verknüpfung verbundenen Glykostruktur. Der tumorspezifische Rezeptor kann in einem Screeningverfahren zur Identifizierung von spezifischen Bindepartnern eingesetzt werden. Spezifische Bindepartner an den Rezeptor sind im Sinne der vorliegenden Erfindung solche Substanzen, die selektiv an eine tumorspezifische Glykostruktur, aber nicht signifikant an eine in normalen Zellen vorkommenden Glykostrukturen von CD55/DAF binden und vorzugsweise die Fähigkeit zur Apoptoseinduzierung besitzen. Diese spezifischen Bindepartner können für die Herstellung von therapeutischen Mitteln zur Apoptoseinduzierung oder/und zur Tumorbekämpfung sowie zur Herstellung von diagnostischen Mitteln eingesetzt werden.

Die Bindung des Antikörpers SC-1 an die tumorspezifische N-verknüpfte Glykostruktur des CD55/DAF-Proteins induziert eine Tyrosinphosphorylierung von drei Proteinen und die Aktivierung von Caspase-3 und Caspase-8. Weiterhin wurde gefunden, daß die durch den Antikörper SC-1 induzierte Apoptose zu einer transienten Zunahme der Präsentation von tumorspezifisch N-glykosyliertem CD55/DAF an der Oberfläche von Tumorzellen führt. Diese erhöhte Präsentation kann durch eine erhöhte Expression oder/und durch eine erhöhte Glykosylierung hervorgerufen werden. Anschließend verschwindet das tumorspezifisch N-glykosylierte CD55/DAF-Protein von der Zellmembran durch Endocytose. Weiterhin wird eine Spaltung von Cytokeratin 18, eine erhöhte Expression von c-myc und eine Abnahme der Expression von Topoisomerase IIα und somit eine mindestens partielle Zellzyklusarretierung beobachtet. Die durch SC-1 induzierten apoptotischen Prozesse führen nicht zu einer erhöhten Spaltung von Poly (ADP-Ribose)-Polymerase (PARP). Weiterhin findet man einen Anstieg der intrazellulären Ca²⁺-Konzentration, das aus einem intrazellulären Ca²⁺-Pool freigesetzt wird. Eine Inhibierung der Ca²⁺-Freisetzung inhibiert die durch SC-1 induzierte Apoptose.

Ein erster Aspekt der Erfindung betrifft ein Glykoprotein umfassend mindestens einen Abschnitt der Aminosäureprimärstruktur von CD55/DAF, insbesondere der membrangebundenen Isoform DAF-B und eine für Tumorzellen spezifische Glykostruktur, insbesondere eine solche Glykostruktur, die mit dem monoklonalen Antikörper SC-1 reagiert. Ein derartiges, beispielsweise aus der humanen Adenokarzinomzellinie 23132 (DSM ACC 201) oder aus anderen humanen Adenokarzinomzellinien, wie 3051 (DSM ACC 270) oder 2957 (DSM ACC 240), oder aus primären Tumorzellen von Magenadenokarzinompatienten erhältliches Glykoprotein weist bei SDS-Polyacrylamid-Gelelektrophorese (unter reduzierenden Bedingungen) ein scheinbares Molekulargewicht von etwa 82 kD auf. Neben diesem 82 kD Protein betrifft die Erfindung auch Varianten mit Deletionen, Insertionen oder/und Substitutionen in der Aminosäureprimärstruktur, die jedoch eine dem natürlichen Protein analoge, d.h. tumorspezifische und vorzugsweise mit dem Antikörper SC-1 reaktive Glykostruktur besitzen.

Das erfindungsgemäße Glykoprotein kann erhalten werden, indem man Membranpräparationen aus Zellen, die ein Protein mit der gewünschten Glykostruktur exprimieren, z.B. aus Zellen der humanen Adenokarzinom-Zellinie 23132 oder aus anderen humanen Adenokarzinomzellinien herstellt und daraus das Glykoprotein durch chromatographische Verfahren z.B. durch Größenausschluß- oder/und Anionenaustauschchromatographie gewinnt. Die Herstellung der Membranpräparationen erfolgt vorzugsweise durch Lyse der Zellen in hypotonischem Puffer, Ultraschallbehandlung und anschließende Abtrennung der Zellkerne. Die Membranpräparationen können durch Zentrifugation aus dem verbleibenden Extrakt isoliert und durch chromatographische Methoden weiter aufgereinigt werden.

Das tumorspezifische CD55/DAF-Glykoprotein kann in einem Testverfahren eingesetzt werden, bei dem die Bindefähigkeit einer Substanz an das tumorspezifische Glykoprotein, insbesondere an dessen Glykostruktur bestimmt wird. Das Testverfahren kann als Hochdurchsatz-Screeningverfahren automatisiert werden. Hierzu kann das Glykoprotein in isolierter Form, als Zellextrakt, insbesondere als Membranpräparation oder in Form vollständiger Zellen, insbesondere der humanen Adenokarzinomzellinie 23132 oder einer anderen humanen Adenokarzinomzellinie, oder einer mit dem CD55-Gen transformierten heterologen eukaryontischen Zelle, z.B. einer Säugerzelle, die in der Lage ist, ein Protein mit der richtigen Glykostruktur zu erzeugen, eingesetzt werden. Als Kontrolle kann die Bindung der getesteten Substanz an ein Nichttumor-CD55/DAF-Glykoprotein untersucht werden, das aus normalen humanen Zellen oder Zellinien erhältlich ist. Substanzen, die selektiv an das tumorspezifische Glykoprotein binden, sind zur Herstellung von therapeutischen oder/und diagnostischen Mitteln geeignet.

Vorzugsweise bestimmt man weiterhin die Fähigkeit der getesteten Substanz zur Apoptoseinduzierung, insbesondere bei Tumorzellen oder/und die Fähigkeit zur Induzierung einer über CD55/DAF vermittelten Phosphorylierungskaskade. Die Induzierung der Apoptose kann durch morphologische Zelluntersuchungen, durch Apoptosetestverfahren, z.B. durch einen Adhäsionstest (Vollmers et al., Cell 40 (1985), 547-557), durch Bestimmung der Keratin 1- und DNA-Fragmentierung, oder durch Proliferationstests wie dem MTT-Proliferationstest durchgeführt werden. Alternativ kann auch eine Bestimmung von Caspase-Aktivitäten, beispielsweise von Aktivitäten von Caspase-8 und/oder Caspase-3 oder eine Bestimmung der intrazellulären freien Calciumkonzentration erfolgen. Substanzen, die selektiv eine Apoptose von Tumorzellen induzieren, können als antitumorwirksame Substanzen eingesetzt werden. Die Induzierung der Phosphorylierungskaskade kann durch Verwendung von für Phosphorgruppen, z.B. Phosphotyrosin- oder/und Phosphoseringruppen spezifischen Antikörpern verfolgt werden.

Zweckmäßigerweise werden pharmakologisch verträgliche Substanzen getestet. Hierzu zählen niedermolekulare pharmakologische Wirkstoffe, insbesondere jedoch Peptide, Peptidmimetika, Antikörper, z.B. polyklonale, monoklonale, oder rekombinante Antikörper, Antikörperfragmente oder Antikörperderivate. Weitere Beispiele für Liganden des CD55/DAF-Rezeptors sind Aptamere (NexStar Pharmaceuticals, 2860 Wilderness Place, Boulder, Colorado 80301, USA) und Spiegelmere (Noxxon Pharma, Gustav-Meyer-Allee 25, 13355 Berlin). Besonders bevorzugt sind beispielsweise rekombinante Antikörper wie etwa einzelkettige scFv-Antikörper, wie sie beispielsweise in Bakterienzellen wie etwa E.coli (Plückthun, Bio/Technologiy 9 (1991), 545-551 und darin zitierte Literaturstellen) oder auch in eukaryontischen Wirtszellen (Reff, Curr. Opinion Biotech. 4 (1993), 573-576 und Trill et al., Curr. Opinion Biotech 6 (1995), 553-560 oder darin zitierte Literaturstellen) erzeugt werden können. Weiterhin bevorzugt sind humane Antikörper, d.h. Antikörper mit humanen konstanten Domänen, wie sie im menschlichen Körper, z.B. von Karzinompatienten, erzeugt werden, oder chimäre und humanisierte Antikörper, bei denen ursprünglich vorhandene nichthumane konstante Domänen oder/und Frameworkregionen durch entsprechende humane Bereiche ausgetauscht wurden. Beispiele für Antikörperfragmente sind Fab-, F(ab)₂- oder Fab'-Fragmente, wie sie durch proteolytische Spaltung von Antikörpern erhalten werden können. Zu den Antikörperderivaten zählen beispielsweise Konjugate von Antikörpern mit Markierungsgruppen oder/und Effektorgruppen, beispielsweise toxischen Substanzen wie etwa Choleratoxin oder Pseudomonas Exotoxin A oder radioaktiven Substanzen.

Substanzen, die spezifisch an das erfindungsgemäße Tumorglykoprotein CD55/DAF binden (mit Ausnahme des bereits bekannten monoklonalen Antikörpers SC-1), können zur Herstellung von die Apoptose-induzierenden Mitteln oder/und zur Herstellung von Antitumormitteln oder/und zur Herstellung von Mitteln zur Tumordiagnostik verwendet werden. Eine tumorspezifische oder tumorselektive Bindung im Sinne der vorliegenden Anmeldung bedeutet vorzugsweise, daß eine Substanz im immunhistochemischen Nachweis mit Tumorzellen, aber nicht signifikant mit anderen Zellen reagiert. Eine Induzierung der Apoptose im Sinne der vorliegenden Anmeldung bedeutet eine Erhöhung des Apoptoseindex, d.h. der Anteil apoptotischer Zellen nach Behandlung mit der Substanz gegenüber den proliferierenden Zellen ist höher als ohne Behandlung, vorzugsweise höher als 50%. Der spontane Apoptoseindex ohne Behandlung liegt deutlich unter 10%, wobei der Nachweis von proliferierenden Zellen über das Antigen Ki67 erfolgen kann.

Mit der vorliegenden Erfindung wird es möglich sein, Verfahren zur Bereitstellung von die Apoptose induzierenden Mitteln oder/und Antitumormitteln oder/und zur Herstellung von Mitteln zur Tumordiagnostik zu entwickeln, wobei man eine potentiell wirksame Substanz auf ihre Fähigkeit zur spezifischen Bindung an ein erfindungsgemäßes Glykoprotein testet und bei einem positiven Testergebnis die Substanz in eine für pharmazeutischen Anwendungen geeignete Darreichungsform gegebenenfalls zusammen mit üblichen Hilfs-, Zusatz- und Trägerstoffen überführt.

Geeignete pharmazeutische Darreichungsformen enthalten den Wirkstoff in einer therapeutisch wirksamen Menge, insbesondere in einer antitumorwirksamen Menge. Die einem Patienten verabreichte Dosis und die Behandlungsdauer hängen von der Art und Schwere der Erkrankung ab. Geeignete Dosierungen für die Verabreichung von Antikörpern sind beispielsweise bei Ledermann et al. (Int. J. Cancer 47 (1991), 659-664) und Bagshawe et al. (Antibody, Immunoconjugates and Radiopharmaceuticals 4 (1991), 915-922) beschrieben.

Der Wirkstoff kann alleine oder in Kombination mit anderen Wirkstoffen entweder gleichzeitig oder sequenziell verabreicht werden. Die pharmazeutische Zusammensetzung kann neben dem Wirkstoff weitere pharmazeutisch übliche Substanzen enthalten. Die Zusammensetzung kann beispielsweise oral, nasal, pulmonal oder durch Injektion verabreicht werden. Oral verabreichbare Zusammensetzungen können in Form von Tabletten, Kapseln, Pulvern oder Flüssigkeiten vorliegen. Durch Injektion verabreichbare Zusammensetzungen sind üblicherweise in Form einer parenteral verträglichen wässrigen Lösung oder Suspension.

Außerdem betrifft die Erfindung die erfindungsgemäßen Glykoproteine zur Bekämpfung von Tumoren.

Ein Gegenstand der Erfindung sind die Glykoproteine zur Diagnose von Tumoren, wobei man eine zu testende Probe, z.B. eine Körperflüssigkeit oder eine Gewebeprobe, oder einen Patienten mit einer an ein tumorspezifisches CD55/DAF Glykoprotein bindefähigen Substanz in Kontakt bringt und das Vorhandensein, die Lokalisierung oder/und die Menge des Glykoproteins in der Probe oder im Patienten nachweist.

Noch ein Gegenstand der Erfindung ist die Verwendung von Substanzen, welche spezifisch das Tumorglykoprotein CD55/DAF binden, zum Auslösen einer Phosphorylierungskaskade. Noch ein weiterer Gegenstand der Erfindung ist die Verwendung von Substanzen, welche spezifisch an das Tumorglykoprotein CD55/DAF binden, zur transienten Erhöhung der Präsentation von Tumorglykoprotein CD55/DAF an der Zelloberfläche, die durch eine erhöhte Glykosylierung oder/und Expression hervorgerufen werden kann. Anschließend verschwindet das tumorspezifische Glykoprotein von der Zelloberfläche. Noch ein weiterer Gegenstand der Erfindung ist die Verwendung von Substanzen, welche selektiv an das Tumorglykoprotein CD55/DAF binden, zur Erhöhung des intrazellulären Calciumspiegels. Substanzen, die spezifisch an das Tumorglykoprotein CD55/DAF binden, können auch als Mittel zur Zellzyklusarretierung eingesetzt werden. Schließlich betrifft die Erfindung auch die Verwendung von Substanzen, die spezifisch an das Tumorglykoprotein CD55/DAF binden, zur Induzierung von apoptotischen Prozessen, die keine Spaltung von PARP umfassen. Die Substanzen können gegebenenfalls als Konjugate mit Markierungs- oder/und Effektorgruppen eingesetzt werden.

Noch ein weiterer Gegenstand der Erfindung ist die Verwendung von Substanzen, die spezifisch an das Tumorglykoprotein CD55/DAF binden, insbesondere des Antikörpers SC-1 zur Induzierung von Apoptose in ruhenden Tumorzellen. Dieser Befund ist nach Kenntnisstand der Erfinder bisher noch für keine tumorselektive Substanz bekannt gewesen.

Die das tumorspezifische Glykoprotein CD55/DAF bindenden Substanzen enthalten vorzugsweise multiple, d.h. mindestens zwei Bindungsstellen für CD55/DAF. Beispielsweise können die Substanzen drei, vier, fünf, sechs, sieben, acht, neun, zehn oder mehr Bindungsstellen enthalten, so daß bei Bindung an zellständiges tumorspezifisches CD55/DAF eine Quervernetzung entsteht. Um Substanzen mit multiplen Bindungsstellen zu erhalten, können Bindemoleküle gegebenenfalls quervernetzt werden. Die Quervernetzung kann beispielsweise mittels chemischer Kopplung, z.B. über bifunktionelle Linkermoleküle, oder über hochaffine Wechselwirkungen, z.B. Streptavidin/Biotin, erfolgen. Auch wenn es sich bei dem CD55/DAF-Bindemolekül beispielsweise um Antikörper, z.B. IgG oder IgM, handelt, die bereits mehrere Bindungsstellen enthalten, kann durch Quervernetzung z.B. über Anti-IgG- oder Anti-IgM-Antikörper noch eine Verbesserung der Apoptoseinduzierung erreicht werden. Die Verwendung von quervernetzten Antikörpern ist daher bevorzugt.

Die Zellinie 23132 ist von der Deutschen Sammlung für Mikroorganismen und Zellkulturen GmbH, Braunschweig, unter dem Aktenzeichen DSM ACC 201 erhältlich.

Weiterhin wird die Erfindung durch die nachfolgenden Beispiele und Figuren erläutert. Es zeigen:
- Figur 1:: die Identifizierung von mit dem Antikörper SC-1 reaktiven Antigenen.
a: Aufreinigung von SC-1 Antigenen aus Membranextrakten der Magenkarzinomzellinie 23132.
b: Sequenzierung eines als SC-1 Antigen identifizierten 82 kD Proteins durch Nanoelektrospray-Tandem-Massenpektroskopie.
- Figur 2:: der Einfluß einer Spaltung von GPI-Ankern durch Phosphatidylinositol-spezifische Phospholipase C (PI-PLC) auf eine Anfärbung mit SC-1. Unbehandelte Magenkarzinomzellen der Zellinie 23132 angefärbt mit SC-1 (a) und Anti-EMA (c); mit PI-PLC behandelte Zellen angefärbt mit SC-1 (b) und Anti-EMA (d) (400 x Vergrößerung).
- Figur 3:: das Ergebnis eines MTT Test mit dem Antikörper SC-1 bei Magenkarzinomzellen. Kontrolle: unbehandelte Zellen; SC-1: mit SC-1 behandelte Zellen; SC-1, PIPLC: mit Phospholipase und anschließend mit SC-1 behandelte Zellen.
- Figur 4:: das Ergebnis einer Analyse von mit einem CD55-Antisense-Vektor transient transfizierten Zellen. Zellen, die mit einem Kontrollvektor transfiziert wurden, zeigen ein normales Anfärbungsmuster mit SC-1 (a) und Anti-CEA (c). In mit dem Antisense-Vektor transfizierten Zellen ist die Anfärbung mit SC-1 verringert (b), während keine Änderung bei Anfärbung mit Anti-CEA (d) zu erkennen ist.
- Figur 5:: das Ergebnis eines Klenow-Fragmentierungstests. Transfizierte Zellen zeigen keine Apoptose ohne Induzierung mit SC-1 (e) im Vergleich zu einer positiven Kontrolle (f). Nach Inkubation mit SC-1 zeigen die mit dem Kontrollvektor transfizierten Zellen Apoptose (g), während die Mehrzahl der mit dem CD55 Antisense-Vektor transfizierten Zellen resistent gegenüber Apoptose ist (h).
- Figur 6:: eine quantitative Bestimmung der durch SC-1 induzierten Apoptose. Mit dem Kontroll- und dem CD-55 Antisense-Vektor transfizierte Zellen wurden mit SC-1 inkubiert und Cytospins dieser Zellen mit dem Klenow DNA Fragmentierungskit angefärbt. Die Prozentzahlen apoptotischer Zellen wurden von zwei verschiedenen Personen durch Zählung Apoptose-positiver und negativer Zellen in drei verschiedenen Feldern mit jeweils etwa 500 Zellen bestimmt.
- Figur 7:: die Wirkung eine Deglykosilierung auf die Bindung des Antikörpers SC-1.
a: Tumorzellen mit Puffer inkubiert und mit SC-1 angefärbt;
b: mit N-Glykosidase und mit SC-1 inkubierte Zellen;
c: mit Puffer und Anti-CD55 inkubierte Zellen und
d: mit N-Glykosidase und Anti-CD55 inkubierte Tumorzellen.
- Figur 8:: Das Ergebnis eines MTT-Tests mit SC-1 bei der Magenkarzinom-Zellinie 23132.
a: Titration von SC-1;
b: Quervernetzung von SC-1 mit Kaninchen-Anti-human IgM-Antikörpern;
- Figur 9:: die Änderung der intrazellulären Calciumkonzentration nach Induzierung der Zellinie 23132 mit SC-1. An Punkt 1 erfolgte die Zugabe von SC-1 bzw. Kontrollantikörper. An Punkt 2 wurden die Zellen mit Ringerlösung gewaschen.
- Figur 10:: das Expressions- und Aktivitätsmuster der Caspasen 3 und 8 nach Induzierung mit SC-1.
a: Westernblot-Analyse der Caspasen-3 und -8. Die Aktivierung von Caspase-3 aufgrund proteolytische Spaltung ist durch Auftreten des p20 Spaltprodukts zu erkennen.
b: das Ergebnis einer Aktivitätsbestimmung von Caspase-8. Ein vierfacher Anstieg der Caspase-8 Aktivität wurde 20 Stunden nach Induzierung der Apoptose gefunden.

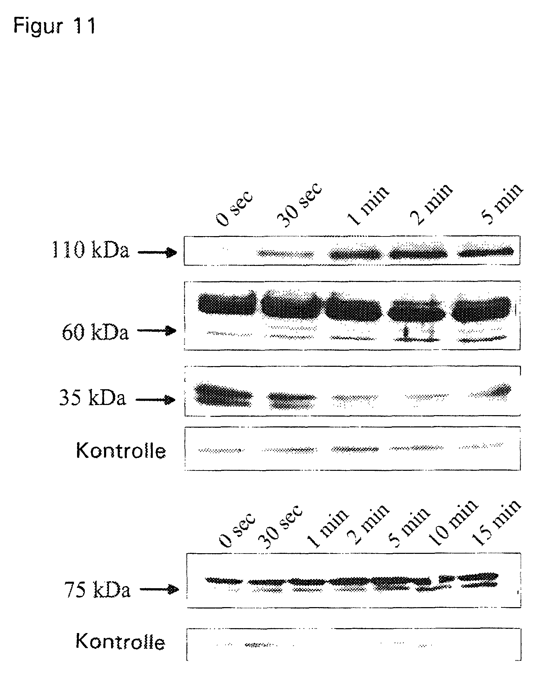

- Figur 11:: die Phosphorylierungsmuster der Zell-Linie 23132 nach Induzierung der Apoptose.
a: Eine rasche Phosphorylierung von Tyrosinresten in Proteinen mit Molekulargewichten von etwa 110 kD und 60 kD sowie die Dephosphorylierung eines Serinrests in einem Protein mit etwa 35 kD wurde nach Induzierung der Apoptose mit SC-1 gefunden.
b: Eine Zunahme der Phosphorylierung eines Thyrosinrestes in einem 75 kD Protein mit einem Maximum nach 10 min wurde nach Induzierung der Apoptose gefunden.

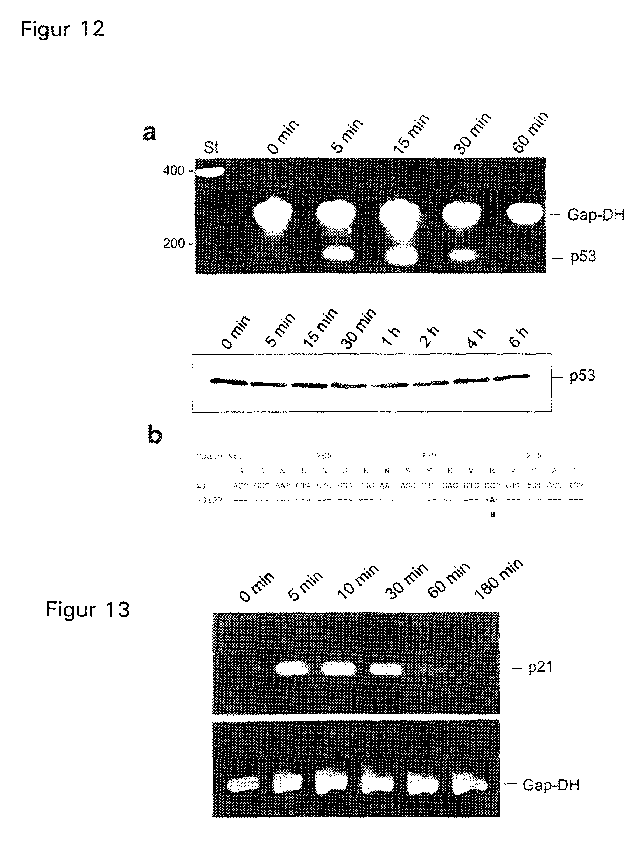

- Figur 12:: eine Expressions- und Mutationsanalyse von p53.
a: 5 min nach Induzierung der Apoptose durch SC-1 wurde eine starke Zunahme der mRNA Konzentration gefunden, während die hohen p53 Proteinkonzentrationen unverändert bleiben.
b: eine Sequenzanalyse von p53 zeigte eine Mutation in Codon 273, die zu einem Aminosäureaustausch von Arg zu His führt.
- Figur 13:: eine Expressionsanalyse von p21.
Nach Induzierung der Apoptose wird eine Zunahme der p21 mRNA Konzentration gefunden.

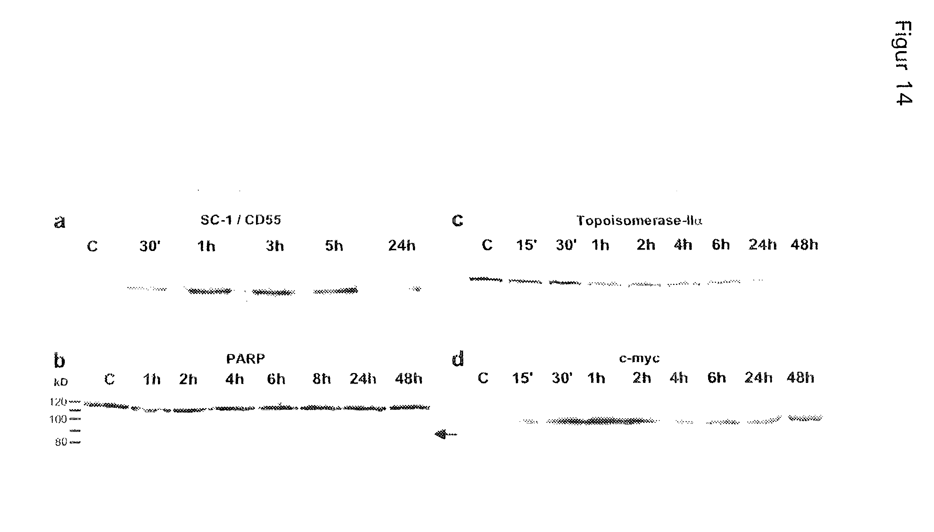

- Figur 14:: eine Western Blot-Analyse von SC-1 induzierten Zellen.
a: CD55/DAF Expression (Anfärbung mit SC-1)
b: Spaltung von PARP (Anfärbung mit Anti-PARP-Antikörper)
c: Anfärbung mit Anti-Topoisomerase IIα-Antikörper als Marker für zelluläre Proliferation
d: c-myc Expression (Anfärbung mit Anti-c-myc-Antikörper)

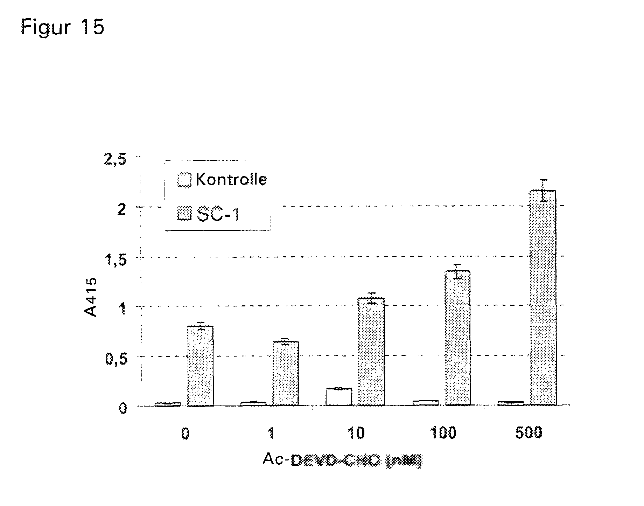

- Figur 15:: die Wirkung des Caspase-3 Inhibitors Ac-DEVD-CHO auf die SC-1-induzierte Apoptose.

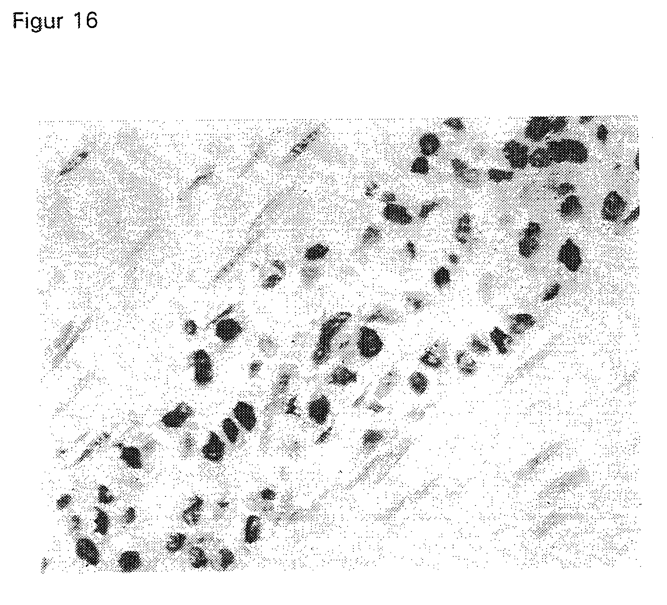

- Figur 16:: den Nachweis einer tumorzellspezifischen Apoptose durch in situ Kernfärbung bewirkt durch Verabreichung des Antikörpers SC-1 an einem Primärtumor.

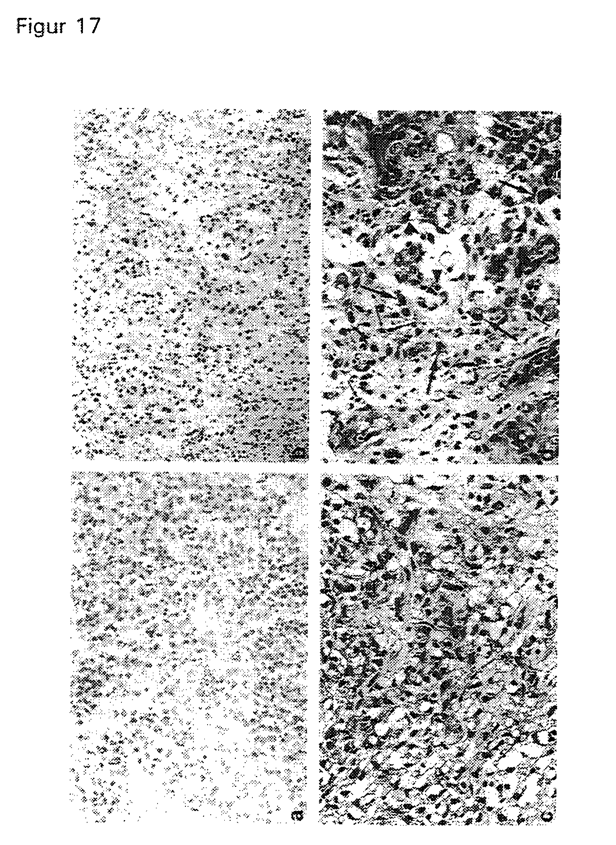

- Figur 17:: die Wirkung der Verabreichung des Antikörpers SC-1 auf einen Primärtumor.
a: Biopsieprobe vor Verabreichung von SC-1 (in situ Färbung auf Apoptose)
b: Primärtumor nach Verabreichung von SC-1 (in situ Färbung auf Apoptose)
c: Biopsie vor Verabreichung von SC-1 (histologische Regressionsanalyse)
d: Primärtumor nach Verabreichung von SC-1 (histologische Regressionsanalyse).

### Beispiele

### 1. Material und Methoden

### 1.1 Zellkultur

Für alle Tests wurde die etablierte Magenadenokarzinomzellinie 23132 (Vollmers et al., Virchows Arch. B. Zell. Pathol. Incl. Mol. Pathol. 63 (1993), 335-343) verwendet. Die Zellen wurden in RPMI-1640 mit 10% fötalem Kälberserum und Penicillin/Streptomycin (beide 1%) bis zur Subkonfluenz kultiviert. Für die beschriebenen Testverfahren wurden Zellen mit Trypsin/EDTA abgelöst und zweimal mit phosphatgepufferter Salzlösung (PBS) vor der Anwendung gewaschen. Die humane Hybridomzellinie SC-1 wurde wie bei Vollmers et al. (Cancer Res. 49 (1989), 2471-2476) beschrieben hergestellt und kultiviert.

### 1.2 Aufreinigung des Antikörpers SC-1

Der humane monoklonale Antikörper SC-1 wurde aus Massenkulturen unter Verwendung von Kationenaustauschchromatographie gefolgt von Gelfiltration wie bei Vollmers et. al. (Oncology Reports 5 (1998), 35-40) beschrieben aufgereinigt.

### 1.3 Aufreinigung des SC-1-Rezeptors

Zur Präparation von Membranproteinen wurden geerntete Zellen in hypotonischem Puffer (20 mM HEPES, 3 mM KCI, 3 mM MgCl₂) resuspendiert, 15 min auf Eis inkubiert und 5 min sonifiziert. Die Zellkerne wurden durch Zentrifugation (10.000 g, 10 min) pelletiert. Die Membranen wurden durch Zentrifugation (30 min, 100.000 g) pelletiert und in Membranlysepuffer (50 mM HEPES, pH 7,4, 0,1 mM EDTA, 1 M NaCl, 10% Glycerin und und 1 % Triton X-100) resuspendiert. Allen Lösungen wurde Complete® Proteaseinhibitor (Boehringer Mannheim, Deutschland) zugesetzt.

Die Aufreinigung der Antigene erfolgte durch Säulenchromatographie unter Verwendung einer FPLC-Einheit (Pharmacia, Freiburg, Deutschland). Für die Größenausschlußchromatographie wurde eine Pharmacia Superdex 200 Säule (XK16/60) mit 5 mg Membranproteinpräparation in Puffer A (100 mM Tris HCl pH 7,5, 2 mM EDTA, 40 mM NaCl, 1 % Triton X-100) beladen. Das Säuleneluat wurde fraktioniert und in einer Westernblot-Analyse auf Reaktion mit dem Antikörper SC-1 untersucht. Positive Fraktionen wurden unter Verwendung von Puffer A auf eine MonoQ-Säule geladen. Die gebundenen Proteine wurden mit einem linearen Gradienten unter Verwendung von Puffer B (100 mM Tris-HCl pH 7,5, 1 M NaCl, 2 mM EDTA, 1 % Triton X100) fraktioniert und durch SDS-Polyacrylamid-Gelelektrophorese und Anfärbung mit Coomassie bzw. Westernblot-Analyse untersucht. Positive Banden wurden aus dem Gel ausgeschnitten und sequenziert.

### 1.4 Präparation von Zell-Lysaten nach Induzierung mit SC-1

Die Zelline 23132 wurde auf 100 mm Zellkulturschalen bis zur Subkonfluenz kultiviert. Der Antköper SC-1 wurde in einer Endkonzentration von 30 µg/ml für die jeweils angegebene Zeitdauer zugegeben. Dann wurden die Kulturplatten einmal mit PBS gewaschen und die Zellen wurden mit SDS-Puffer (50 mM Tris-HCl pH 6,8, 10 mM Dithiothreitol, 2% (w/v) SDS, 10% (v/v) Glycerin) direkt lysiert. Die Zellrückstände wurden mit einem Gummischaber gesammelt.

### 1.5 Gelelektrophorese und Blots

Die SDS-Polyacrylamid-Gelelektrophorese unter reduzierenden Bedingungen und das Western-Blotting von Proteinen wurde unter Verwendung von Standardprotokollen wie bei Vollmers et al. (Cancer 79 (1997), 433-440) beschrieben durchgeführt. Nitrozellulosemembranen wurden mit PBS unter Zusatz von 0,1 % Tween-20 und 2% Magermilchpulver oder 3 % Rinderserumalbumin (zur Bestimmung der Phosphorylierung) blockiert und anschließend eine Stunde lang mit dem Primärantikörper inkubiert. Die Antikörper wurden in folgenden Verdünnungen eingesetzt. SC-1 (human) 10 µg/ml bzw. 15 µg/ml; Anti-Caspase-3 bzw. -8 (Ziege) (SantaCruz, Heidelberg, Deutschland) 5 µg/ml, Streptavidin Anti-Phosphotyrosin-Konjugat (Klon PT-66) 1:20.000 und Streptavidin Anti-Phosphoserin-Konjugat (Klon PSR-45) 1 :30.000 (Sigma, München, Deutschland), Maus-Anti-Topoisomerase IIα-Antikörper 1:1.000 (Neomarkers, Baesweiler, Deutschland), Anti-c-myc-Antikörper 1:1.000 (Santa Cruz, Heidelberg, Deutschland) und Anti-PARP-Antikörper 1:1.000 (Pharmingen, Heidelberg, Deutschland). Die Sekundärantikörper Peroxidase-Kaninchen-Anti-Human-IgM-Konjugat oder Kaninchen-Anti-Ziegen-Antikörper (Dianova, Hamburg, Deutschland) und Peroxidasekonjugiertes Extravidin (Sigma) wurden mit dem SuperSignal Chemilumineszenzkit von Pierce (KMF, St. Augustin, Deutschland) nachgewiesen.

### 1.6 Proteinsequenzierung

Eine Proteinbande mit einem scheinbaren Molekulargewicht von 82 kD wurde durch eindimensionale Polyacrylamidgelelektrophorese isoliert und durch Anfärbung mit Coomassie sichtbar gemacht. Die p82-Bande wurde im Gel mit Trypsin (Boehringer Mannheim, nichtmodifiziert, Sequenzierungsqualität) wie bei Shevchenko et al., (Anal. Chem. 68 (1996), 850-858) beschrieben gespalten. Dernichtaufgetrennte Pool vontryptischen Peptiden wurdedurch Nanoelektrospray-Tandem-Massenspektrometrie wievon Wilm et al. (Nature 379 (1996), 466-469) beschrieben sequenziert. Die Sequenzierung erfolgte auf einem API III Triple Quadrupol Massenspektrometer (PE Sciex, Ontario, Kanada). Die Sequenzen der Peptidfragmente wurden unter Verwendung der Tandem-Massenspektrometriedaten assembliert und durch Datenbankrecherchen den jeweiligen Proteinen zugeordnet.

### 1 .7 RT-PCR

Die cDNA-Synthese aus Gesamt RNA der Tumorzellen 23132 erfolgte mit 5 µg Gesamt RNA unter Verwendung von M-MLV Reverser Transkriptase (Gibco BRL, Eggenstein, Deutschland) gemäß den Angaben des Herstellers. Die PCR-Reaktionen wurden in einem Reaktionsvolumen von 25 µl mit 1,75 mM MgCl₂, 0,4 pM Primer, 200 µM von jedem dNTP und 1 U Taq Polymerase (MBI Fermentas, St. Leon-Rot, Deutschland) durchgeführt.

### Es wurden folgende PCR-Produkte erzeugt:

CD55 (640 bp Fragment aus dem Sequenzbereich von bp 382 bis 1022) p53-Fragment 1 (850 bp Fragment aus dem Sequenzbereich von 91 bis 940) p53-Fragment 2 (800 bp aus dem Sequenzbereich von 492 bis 1294)

### 1.8 Klonierungsprozeduren

Die PCR-Produkte wurden aus einem Agarosegel unter Verwendung des Jetsorb Gelextraktionskits (Genomed, Bad Oeynhausen, Deutschland) aufgereinigt. Die Klonierung der PCR-Fragmente erfolgte mit dem pCR-Script Amp SK (+) Klonierungskit (Stratagene, Heidelberg, Deutschland).

Die Klonierung des Antisense-Vektors pHOOK2-CD55-anti erfolgte durch Glätten des CD55-PCR-Produkts mit Pfu-Polymerase und Klonierung in den mit Smal geschnittenen Expressionsvektor pHOOK2 (Invitrogen, Leek, Niederlande). Ein Klon mit Antisense Richtung der Insertion unter Kontrolle des P_{CMV}-Promotors wurde für die Antisense-Experimente ausgewählt.

### 1.9 DNA-Seguenzierung

Acht positive Klone wurden unter Verwendung des DyeDeoxy Termination Cycle Sequencing Kit (Applied BioSystems Inc., Weiterstadt, Deutschland) sequenziert und dem automatisierten DNA Sequenzer ABIPrism 373 analysiert. Beide Stränge wurden unter Verwendung von T3 und T7 Primern sequenziert. Die Sequenzen wurden unter Verwendung der Computerprogramme DNASIS und BLAST analysiert.

### 1 .10 Transfektion

Für Transfektionsexperimente wurden 2-5 x 10⁷ abgelöste Zellen in Trisgepufferter Salzlösung (TBS) gewaschen und in 400 µl TBS resuspendiert. Nach Zugabe von 10 µg Plasmid DNA wurden die Zellen mit 240 V, 960 nF unter Verwendung eines Elektroporationsgeräts von BioRad (München, Deutschland) gepulst. 5 x 10⁵ transfizierte Zellen wurden auf einer 60 mm Zellkulturschale ausgesät und für 24 h wie zuvor beschrieben inkubiert. Die Apoptose wurde durch Zugabe von 50 µg/ml gereinigtem SC-1 Antikörper zum Wachstumsmedium induziert. Nach 24 h wurden die Zellen mit Trypsin behandelt und zur Herstellung von Cytospins verwendet.

### 1.11 Phospholipasetest

Abgelöste und deletierte Zellen wurden RPMI-1640 mit Zusätzen resuspendiert und für 90 min bei 37°C inkubiert. Nach dieser Erholungsperiode wurden 20 mU/ml PI-PLC (Boehringer Mannheim) zugegeben, und die Zellen für weitere 60 min inkubiert. Schließlich wurden die Zellen gewaschen und zur Herstellung von Cytospins verwendet.

### 1.12 Glycosidase-Test

Abgelöste und gewaschene Zellen wurden in RPMI-1640 mit 10% fötalem Kälberserum resuspendiert, 1 h auf Eis inkubiert, dann gezählt und Cytospins hergestellt. Nach Lufttrocknung wurden die Cytospinpräparationen mit Aceton fixiert (10 min), gewaschen und mit 20 µU/ml O-Glykosidase oder 5 mU/ml N-Glycosidase (Boehringer Mannheim) für 4 h bei 37°C inkubiert.

### 1.13 Immunhistochemische Anfärbung

Folgende Antikörper wurden für die immunhistochemische Anfärbung verwendet: Gereinigter Antikörper SC-1, Anti-CEA-Antikörper (DAKO, Hamburg, Deutschland) Anti-EMA-Antikörper (Loxo, Dossenheim, Deutschland) und Anti-CD55-Antikörper (Biozol, Eiching, Deutschland). Die Acetonfixierung und Anfärbung der Cytospinpräparationen erfolgte wie von Vollmers et al. (Hum. Antibodies Hybridomas 7 (1996), 37-41) beschrieben.

Zur immunhistochemischen Anfärbung von apoptotischen Zellen wurden bis zur Subkonfluenz kultivierte Zellen mit gereinigtem Antikörper SC-1 (verdünnt auf 50 µg/ml) in vollständigem Wachstumsmedium für bis zu 96 h inkubiert. Adhärente und abgelöste Zellen wurden gesammelt, zentrifugiert und in vollständigem Wachstumsmedium resuspendiert. Nach einer Zellzählung wurden Cytospin-Präparate hergestellt und bei Raumtemperatur über Nacht getrocknet. Untersuchung der Spaltung von Cytokeratin 18 in vivo wurden Biopsien von Patienten vor Behandlung mit SC-1 und Gewebeschnitte nach Behandlung und Gastrektomie wie bei Vollmers et al., (Oncol. Rep. 5 (1998), 549-552) beschrieben entnommen.

Die Cytospins wurden mit Rinderserumalbumin (15 mg/ml) in phosphatgepufferter Salzlösung (PBS) für 30 min blockiert. Anschließend erfolgte eine Inkubation für 1 h mit SC-1-Überstand, M30 Cyto Death-Antikörper (Roche Biochemicals, Mannheim, Deutschland) oder Maus-Anti-Cytokeratin 18 Antikörper (DAKO, Hamburg, Deutschland) 1: 15 verdünnt. Anschließend wurde für 30 min in PBS gewaschen, gefolgt von einer Inkubation mit Peroxidase-markiertem Kaninchen-Anti-Maus-oderKaninchen-Anti-Human-Konjugat (DAKO) 1:25 verdünnt. Nach 30-minütigem Waschen mit PBS erfolgte die Anfärbung mit Diaminobenzidin (0,05 %) und Wasserstoffperoxid (0,02 %) für 3 min bei Raumtemperatur. Die Reaktion wurde mit Leitungswasser gestoppt und die Gewebeschnitte wurden mit Hämatoxylin gegengefärbt.

### 1.14 Apoptosetests

Cytospinpräparationen (5000 Zellen/Objektträger) wurden in Aceton fixiert und dann mit TBS gewaschen. Anschließend wurden sie mit dem FragE1-Klenow DNA-Fragmentierungskit (Calbiochem-Novabiochem, Bad Soden, Deutschland) nach Angaben des Herstellers angefärbt.

Ein ELISA zum Nachweis der Apoptose wurde unter Verwendung des Cell Death Detection® Kit (Roche Biochemicals) nach der Vorschrift des Herstellers durchgeführt.

### 1.15 MTT-Test

Der MTT-Proliferationstest (Carmichael et al., Cancer Res. 47 (1987), 936-942) zur Bestimmung der Apoptoseaktivität des Antikörpers SC-1 auf Magenkarzinomzellen wurde wie bei Vollmers et al. (Cancer 76 (1995), 550-558) beschrieben durchgeführt. Die Bestimmung des Zellwachstums erfolgte durch den mitochondrialen Hydroxylase-Test (Mossmann, J. Immunol. Meth. 65 (1983), 55-63). Aus der Absorption der mit SC-1 induzierten Zellen und der nicht mit SC-1 induzierten Kontrolle wurde der prozentuale Anteil von apoptotischen Zellen bestimmt (Vercammen et al., J. Exp. Med. 188 (1998), 919-930).

### 1.16 Caspase-3 und -8 Tests

Die Aktivierung von Caspase-8 und Caspase-3 wurde mit dem ApoAlert™ Caspase Fluoreszenz-Testkit (Clontech, Heidelberg, Deutschland) bestimmt. Hierzu wurden 1 x 10⁶ Zellen mit 40 µg/ml SC-1 für 7 bzw. 20 h inkubiert.

Dann wurden die Zellen gesammelt, in Zell-Lysepuffer resuspendiert und die Caspaseaktivität nach Angaben des Herstellers bestimmt.

### 1.17 Bestimmung von intrazellulärem freien Calcium [Ca²⁺].

Die Bestimmung der intrazellulären freien Calciumkonzentration wurde unter Verwendung des Calcium-sensitiven Farbstoffs Fura-2-AM wie von Grykiewicz et al. (J. Biol. Chem. 260 (1985), 3440-3450) beschrieben bestimmt. Hierzu wurden die Zellen mit einer Fura-2-AM in einer Endkonzentration von 5 x 10⁻⁶ M enthaltenden Ringerlösung (122,5 mM NaCl, 5,4 mM KCI, 1,2 mM CaCl₂, 0,8 mM MgCl₂, 1 mM NaH₂PO₄, 5,5 mM Glucose, 10 mM HEPES pH 7,4) für 15 min inkubiert. Nach Spülen wurden die Objektträger mit einem Axiovert 100 TV Mikroskop (400-fache Vergrößerung) untersucht. Das Fluoreszenzsignal wurde bei 500 nm mit zwischen 334 und 380 nm alternierenden Anregungswellenlängen unter Verwendung einer 100-W Xenon-Lampe und einer automatischen Filterwechselvorrichtung (Zeiss, Deutschland) gemessen. Die Konzentration von intrazellulärem freiem Calcium wurde nach der Methode von Grynkiewicz et al. (supra) unter Annahme einer Dissoziationskonstante von 225 nmol/l berechnet. Die maximalen und minimalen Fluoreszenzverhältnisse (Rₘₐₓ und Rₘᵢₙ) wurden nach Zugabe von Kalibrierungslösungen gemessen. Rₘₐₓ wurde nach Zugabe einer Ringerlösung mit 3 mM Ca²⁺ und 10⁻⁶ M lonomycin bestimmt. Rₘᵢₙ wurde in Gegenwart einer Ca²⁺ freien Ringerlösung mit 3 mM EGTA und 10⁻⁶ M lonomycin bestimmt.

### 1.18 Inhibierung der intrazellulären Calciumfreisetzung

Zellen wurden einmal mit phosphatgepufferter Salzlösung gewaschen und für 24 h in Calcium-freiem DMEM-Medium ohne fötalem Kälberserum (FCS) gewaschen. Dann wurde aufgereinigter SC-1-Antikörper bis zu einer Endkonzentration von 40 µg/ml zugegeben. Als Kontrolle wurden die gleichen Zellen ohne SC-1 verwendet. Die Zellen wurden in einem befeuchteten Inkubator für weitere 24 oder 48 h inkubiert und dann mit 3 % Glutaraldehyd fixiert. Die Zellkulturplatten wurden dann mit Hilfe eines Lichtmikroskops auf morphologische Veränderungen untersucht.

### 2. Ergebnisse

### 2.1 Aufreinigung des SC-1-Rezeptors CD55

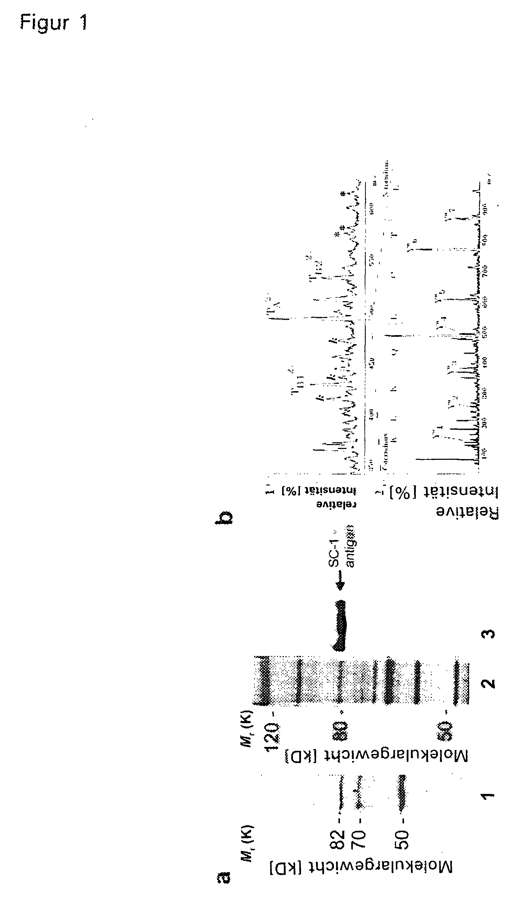

Bei Westernblot-Analyse von Extrakten aus Gesamtzell-Lysaten der Magenkarzinomzellinie 23132, die unter Niedrigsalzbedingungen (100 mM NaCl) hergestellt worden waren, reagierte der Antikörper SC-1 mit einem Protein mit einer relativen Molekularmasse von etwa 50 kD. Durch Änderung der Stringenz (1 M NaCl) und unter Verwendung von Membranpräparationen konnten weitere Proteine mit etwa 70 kD und etwa 82 kD nachgewiesen werden (Figur 1a, Spur 1). Diese Proteine wurden aus Membranfraktionen isoliert und durch sequenzielle Größenausschluß- und Anionenaustauschchromatorgraphie gereinigt (Figur 1a, Spuren 2, 3). Die Moleküle wurden aus SDS-Polyacrylamidgelen ausgeschnitten und sequenziert.

Das 50 kD Protein wurde als Dihydrolipoamidsuccinyltransferase (Genbank-Zugriffsnr. L37418) und das 70 kD Protein als das humane Lupus p70 Autoantigenprotein (Genbank-Zugriffsnr. J04611) identifiziert. Bei diesen Proteinen handelt es sich um zytoplasmatische bzw. nukleäre Antigene. Da der Antikörper SC-1 in immunhistochemischen Untersuchungen nur an Zelloberflächenantigene bindet, ist die Reaktivität vermutlich auf unspezifische Bindung aufgrund der Proteindenaturierung während der Westernblot-Analyse zurückzuführen.

Das 82 kD Protein wurde als CD55 (DAF, Genbank-Zugriffsnr. M31516, Figur 1b, Abschnitte 1 und 2) identifiziert. CD55 existiert beim Menschen in zwei genetisch bestimmten Isoformen (sekretiertes DAF-A und membrangebundenes DAF-B) die durch differentielles Spleißen erzeugt werden (Caras et al., Nature 325 (1987), 545-549). Durch RT-PCR-Analyse wurde festgestellt, daß die Zellinie 23132 nur die membranverankerte DAF-B Isoform exprimiert.

### 2.2 Phospholipase-Behandlung

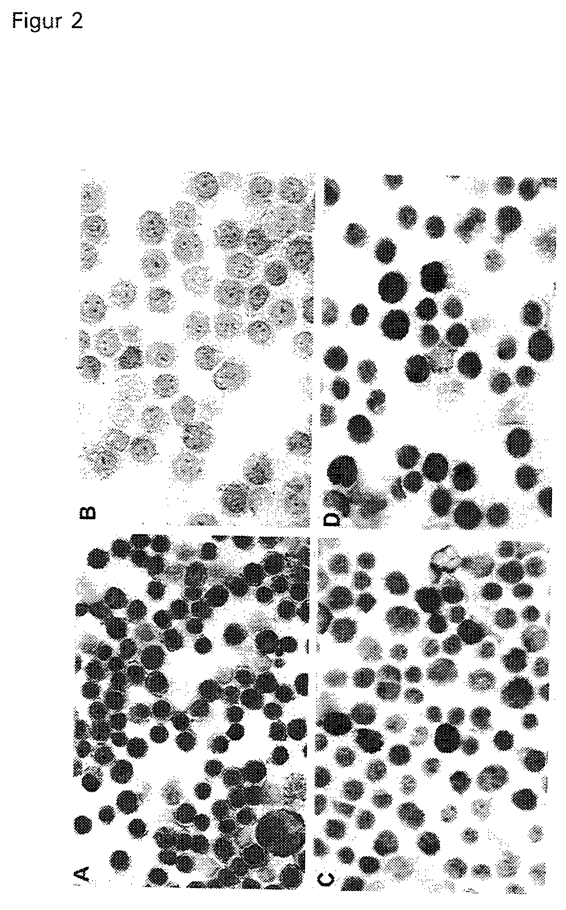

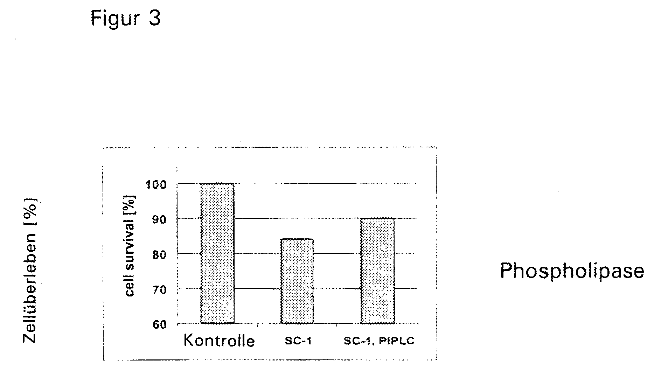

Durch immunhistochemische Untersuchungen und im MTT-Proliferationstest wurde der Einfluß einer Abspaltung des Glykosidphosphatidylinositol (GPI)-Ankers auf die Bindung von SC-1 analysiert. Hierzu wurde der GPI-Anker durch Inkubation mit Phosphatidylinositol-spezifischer Phospholipase C (PI-PLC) abgespalten. Cytospins von mit PI-PLC behandelten und unbehandelten Zellen wurden immunhistochemisch mit SC-1, Anti-CD55 und Anti-EMA (Epithelialmembran-Antigen) angefärbt. Ein Vergleich mit unbehandelten Zellen (Figur 2a) zeigt einen Verlust der Anfärbungsintensität bei mit PI-PLCbehandelten und SC-1 angefärbten Zellen (Figur 2b). Bei Anfärbung mit Anti-EMA (Figur 2c, d) wurde kein Unterschied in der Anfärbung gefunden, was darauf hinweist, daß die PI-PLC-Behandlung keine Wirkung auf nicht GPI-verankerte Membranproteine hat. Beim MTT-Test führte eine Behandlung von Zellen mit Phospholipase C zu einer signifikanten Abnahme (p ≤ 0,05) der apoptotischen Zellen (Figur 3).

### 2.3 Transfektion mit Antisense-CD55 RNA

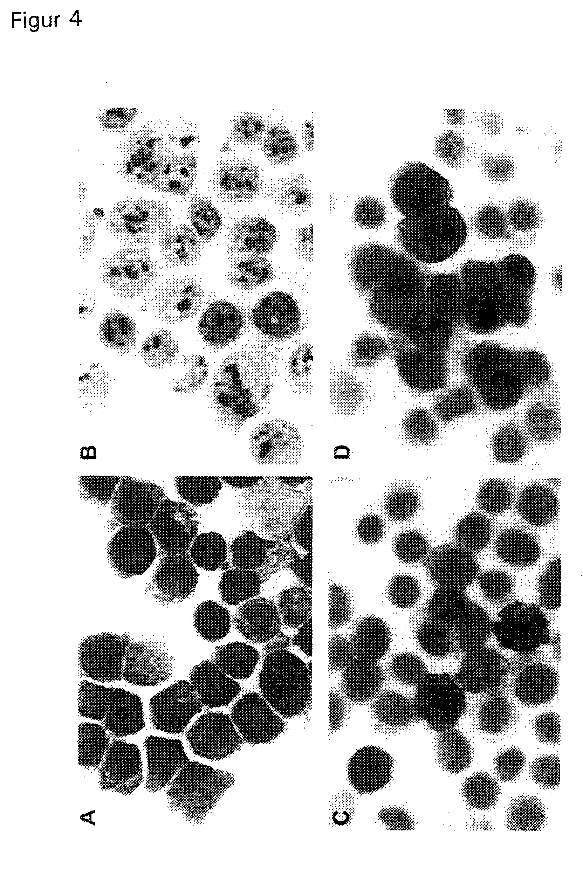

Die Zellinie 23132 wurde mit dem CD55 Antisense-Vektor pHOOK2-CD55anti und dem Kontrollvektor pHOOK2 durch Elektroporation transient transfiziert. Zuerst wurden Cytospins von transfizierten Zellen immunhistochemisch mit SC-1, Anti-CD55 und Anti-CEA (Carcino-embryonales Antigen) angefärbt. Die mit dem Kontrollvektor transfizierten Zellen zeigten eine intensive Anfärbung mit SC-1 und CEA (Figur 4a, c). Bei mit dem CD55 Antisense-Vektor angefärbten Zellen wurde fast keine Anfärbung mit SC-1 gefunden (Figur 4b). Die Anfärbung mit Anti-CEA-Antikörper zeigte, daß das Expressionsmuster des (auch GPI-verankerten) CEA nicht durch die Transfektion mit dem Antisense-Vektor beeinflußt wird. Folglich wurde die Expression von CD55 spezifisch aufgrund der Expression der Antisense RNA reduziert.

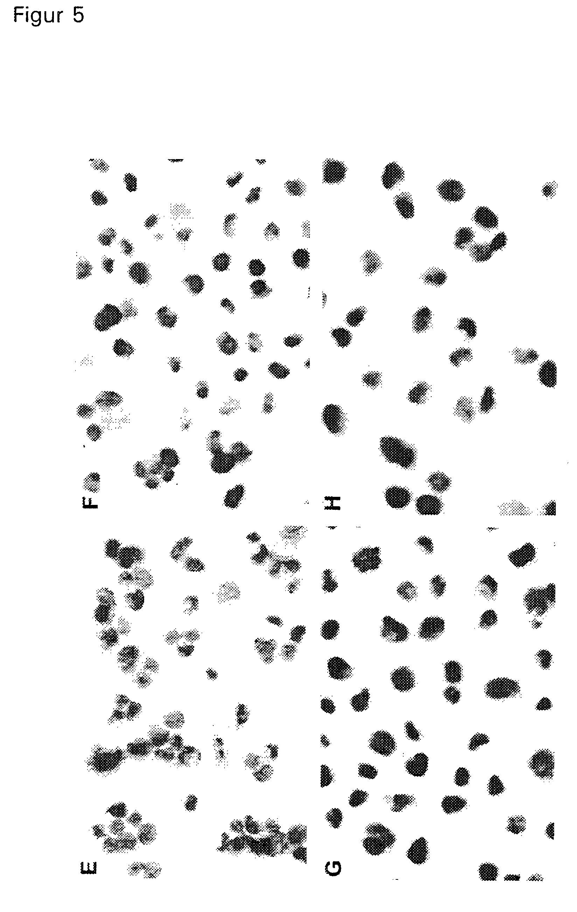

Um zu analysieren, ob die Expression von Antisense-CD55 RNA auch die SC-1 induzierte Apoptose hemmt, wurden die Zellen einen Tag nach der Transfektion mit und ohne 30 µg/ml SC-1 für eine Dauer von 24 h inkubiert. Cytospins von mit dem Antisense-Vektor und dem Kontrollvektor transfizierten Zellen wurden mit dem FragE1 Klenow DNA Fragmentierungskit zum Nachweis einer durch Apoptose induzierten DNA-Fragmentierung angefärbt. Während mit beiden Plasmiden behandelte untransfizierte Zellen nahezu keine spontante Apoptose zeigen (Figur 5e), findet man nach Inkubation mit SC-1 eine deutliche Abnahme bei der Apoptose von mit dem CD55 Antisense-Vektor transfizierten Zellen (Figur 5g) im Vergleich zu mit dem Kontrollvektor transfizierten Zellen (Figur 5h).

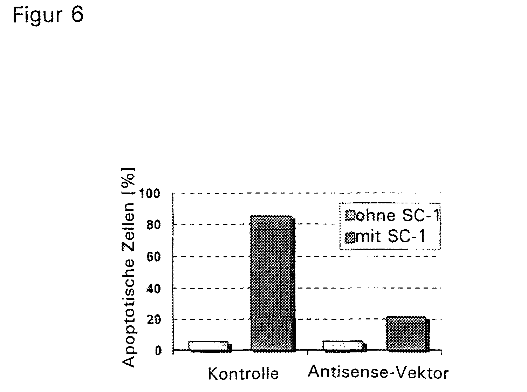

Eine quantitative Bestimmung zeigte, daß spontane Apoptose in transfizierten 23132 Zellen mit einer Häufigkeit von 6% auftrat, während nach Inkubation mit SC-1 85% der mit dem Kontrollvektor transfizierten Zellen eine Apoptose zeigten. Diese apoptotische Reaktion wurde durch Transfektion mit dem CD55 Antisense-Vektor auf 21 % verringert (Figur 6).

### 2.4 Glykosidasebehandlung

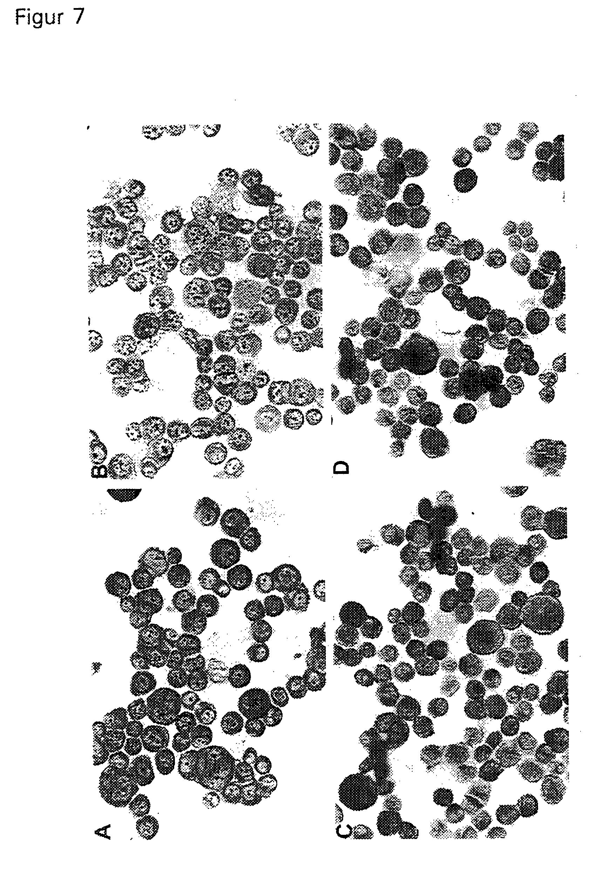

Der Einfluß einer Proteindeglykosilierung auf die Bindung von SC-1 an die Zell-Linie 23132 wurde durch Inkubation von Cytospin-Präparaten mit O-und N-Glykosidasen vor der immunhistochemischen Anfärbung untersucht. Eine Behandlung von Zellen mit N-Glykosidase führte zu einer signifikanten Abnahme der SC-1 Anfärbung (Figur 7b), während eine Anfärbung mit Anti-CD55, der den Proteinanteil in der SCR3 Region erkennt, nicht durch Proteindeglycosilierung beeinflußt wurde (Figur 7d). Eine Inkubation mit Phosphatpuffer und eine Behandlung mit O-Glycosidase hatte keine Wirkung auf die SC-1 Bindung. Dies zeigt, daß die Spezifität von SC-1 in N-verknüpften Zuckerresten und nicht in der Primärproteinsequenz lokalisiert sein muß.

### 2.5 Quervernetzung von CD55/SC-1

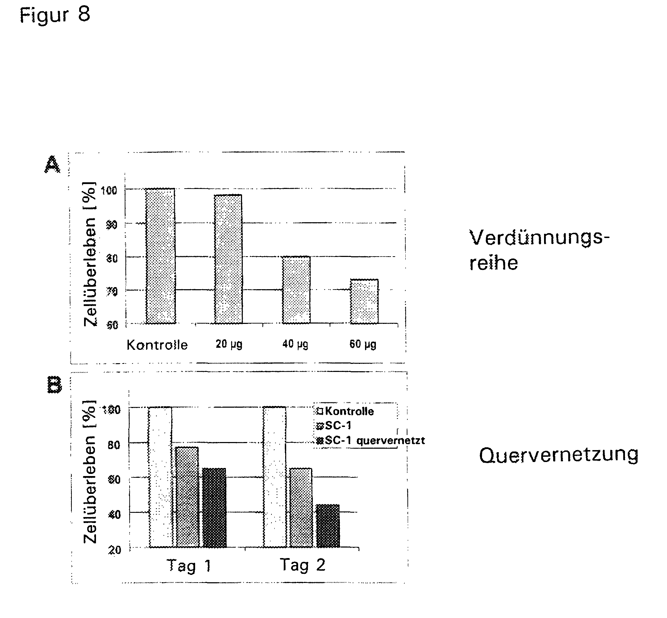

Die Zellen wurden 24 h mit zunehmenden Mengen an SC-1 inkubiert um die optimale apoptopische Aktivität von SC-1 zu bestimmen (Figur 8a). Dann erfolgte Quervernetzung bei einer Konzentration von 40 µg/ml SC-1 mit Kaninchen-Anti-Human IgM. Nach Inkubation für 48 h wurde ein 47% höherer Anteil an toten Zellen als bei mit SC-1 inkubierten Kontrollzellen gefunden (Figur 8b).

### 2.6 Calciumspiegel

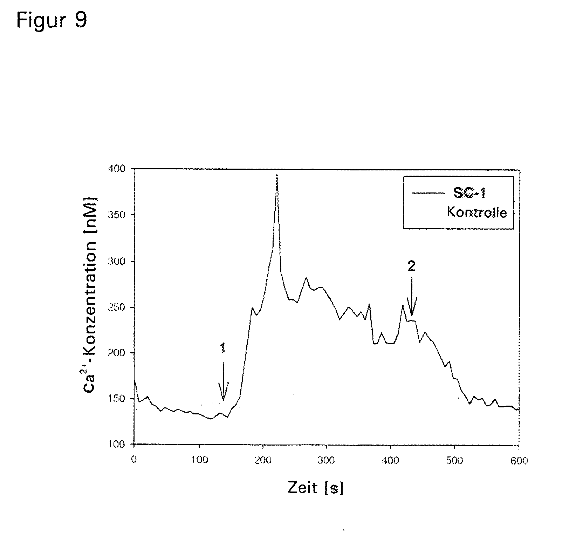

Um zu untersuchen, ob die durch SC-1 induzierte Apoptose mit Änderungen des Calciumspiegels einhergeht, wurde die intrazelluläre Calciumkonzentration der Zellinie 23132 nach Induktion mit SC-1 und Kontrollantikörper (unspezifisches humanes IgM) bestimmt. Dabei konnte ein signifikanter Anstieg der intrazellulären Calciumkonzentration etwa 1 min nach Zugabe des SC-1 Antikörpers gefunden werden, während der Kontrollantikörper keinen Einfluß hatte (Figur 9).

### 2.7 Caspase-Aktivität

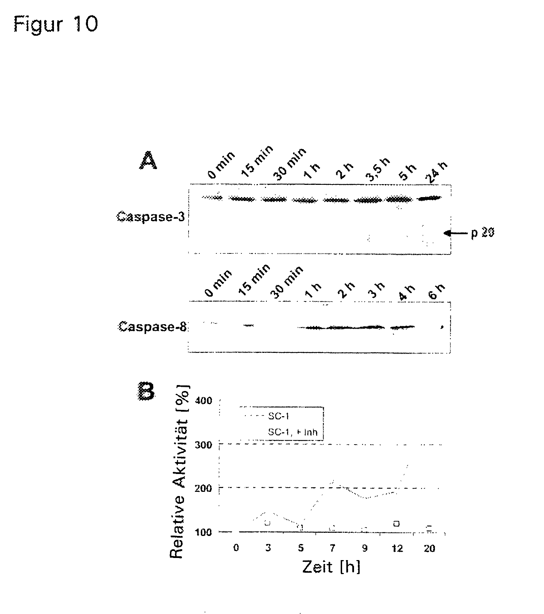

Durch Westerblot-Analyse wurde gefunden, daß die Caspasen-3 und -8 nach Induzierung der Zellinie 23132 mit SC-1 nach oben reguliert werden (Figur 1 0a). Eine die Aktivierung von Caspasen hervorrufende proteolytische Spaltung wurde für Caspase-3 durch Identifzierung des Spaltprodukts p20 nachgewiesen (Figur 10a). Bei Caspase-8 wurde ein vierfacher Anstieg der Aktivität 20 h nach der Induzierung mit SC-1 gefunden, was auf eine wesentliche Beteiligung dieser Caspase beim Apoptose-Prozess hinweist (Figur 10b).

Die Zugabe des spezifischen Caspase-3-Inhibitors AC-DEVD-CHO (Alexis Biochemicals, Grünberg, Deutschland) zeigte überraschenderweise mit steigender Konzentration eine Zunahme der Apoptose bei Bestimmung mit dem Cell Death Detection® Kit (Figur 15).

### 2.8 Proteinphosphorylierung

Das Phosphorylierungsmuster nach Induzierung der Zellen mit 40 µg/ml SC-1 Antikörper wurde durch Westernblot-Analyse von cytoplasmatischen und Membranextrakten untersucht. Dabei wurde eine frühe Tyrosinphosphorylierung von 110 kD und 60 kD Proteinen 30 bis 60 sec nach induzierung der Apoptose gefunden (Figur 11). Das 60 kD Protein wurde nur im Cytoplasma gefunden, während das 110 kD Protein sowohl im Plasma als auch im Membranextrakt nachweisbar war. Weiterhin wurde eine langsame Tyrosinphosphorylierung eines cytoplasmatischen 75 kD Protein mit einem Maximum nach 10 min sowie das vollständige Verschwinden der Serinphosphorylierung eines 35 kD Proteins 10 min nach Induzierung gefunden.

### 2.9 Expression und Sequenzierung von p53

Um die Rolle von p53 bei SC-1 induzierter Apoptose zu untersuchen, wurde die Häufigkeit der mRNA durch RT-PCR und des Genprodukts durch Westernblot-Analyse nach Induzierung bestimmt. Dabei wurde ein deutlicher Anstieg der mRNA-Konzentration gefunden. Auf Proteinebene wurde jedoch eine konstante und nicht signifikant geänderte hohe Konzentration des p53 Genprodukts gefunden (Figur 12a).

Durch Amplifizierung von zwei p53 Fragmenten aus cDNA mit spezifischen Primern, Klonierung der PCR-Fragmente und Sequenzierung von acht Klonen wurde die DNA-Sequenz von p53 in der Zellinie 23132 bestimmt. Alle Klone mit das Exon 8 überspannenden Insertionen zeigten eine Mutation in Kodon 273, die zu einem Aminosäurenaustausch von Arginin zu Histidin führte (Figur 12b). Dies ist eine dominant negative Mutation, die häufig in Magenadenokarzinomen auftritt.

### 2.10 Expression von p21

Das Protein p21 ist ein mit der Expression von p53 assoziiertes Molekül. Ein Test der Expression von p21 in der Magenkarzinomzellinie 23132 nach Behandlung mit SC-1 ergab einen Anstieg nach 5 min gefolgt von einer Verringerung nach 60 min (Figur 13).

### 2.11 Expression von CD55/DAF nach Induzierung der Apoptose

Es wurde das Expressionsmuster von CD55/DAF nach Induzierung der Apoptose durch 50 µg/ml SC-1 mittels immunhistochemischer Anfärbung von Cytospin-Präparationen mit dem Antikörper SC-1 untersucht. Während nichtinduzierte Zellen eine leichte Membrananfärbung mit dem Antikörper SC-1 aufweisen, zeigten mit SC-1 induzierte Zellen eine intensive Membrananfärbung 12 h nach Induzierung der Apoptose. Dies weist auf eine Erhöhung der CD55/DAF-Präsentation an der Zelloberfläche nach Bindung der Antikörper an die Zellen hin. Diese Membrananfärbung verschwindet nach 48 h, und eine diffuse cytoplasmatische Anfärbung ist erkennbar. Diese Anfärbung wird auch nach 96 h mit verringerter Intensität gefunden. Die Zunahme der CD55/DAF-Expression wurde auch bei einer Western Blot-Analyse mit Membranextrakten von apoptotischen Zellen nach SC-1-Induzierung gefunden. Während in nichtinduzierten Zellen CD55/DAF nicht nachweisbar ist, nimmt die CD55/DAF-Expression 1 h bis 6 h nach Induzierung zu. Nach 24 h nimmt die Expression von CD55/DAF ab, ist aber immer noch höher als in nichtinduzierten Zellen (Figur 14a).

### 2.12 Spaltung von Cytokeratin 18

Der Abbau von apoptotischen Zellen geht einher mit der proteolytischen Spaltung von Cytokeratin 18. Die Spaltung von Cytokeratin 18 in der Zellinie 23132 nach SC-1 induzierter Apoptose sowie in Primärtumoren von Patienten, die mit 20 mg SC-1 48 h vor einer Tumorresektion behandelt worden waren, wurde untersucht. Eine M30 Cyto Death-Anfärbung zeigte eine geringe Menge apoptotischer Zellen ohne Induzierung von Apoptose, während die Anzahl apoptotischer Zellen bis 96 h zunimmt.

### 2.13 Molekulare Analyse der SC-1-Apoptose

Im Einklang mit den immunhistochemischen Anfärbungen zeigte die biochemische Analyse eine Zunahme der CD55/DAF-Moleküle, gefolgt von einer leichten Abnahme nach 24 h Inkubation mit SC-1 (Figur 14a). Die Spaltung von PARP wurde durch Western Blot-Analyse unter Verwendung von Gesamtzellextrakten aus SC-1 induzierten Zellen und murinem Anti-PARP-Antikörper untersucht. In fünf unabhängigen Experimenten wurde keine Spaltung von PARP gefunden, die sich durch das Auftreten eines 85 kD Spaltungsprodukts (Lazebnik et al., Nature 371 (1994), 346-347) nachweisen lassen würde (Figur 14b).

Um Veränderungen im Zellzyklus nach Induzierung der Apoptose zu untersuchen, wurde die Expression der Topoisomerase IIα durch Western Blot-Analyse bestimmt. Die Topoisomerase IIα ist ein Schlüsselenzym im Zellzyklus, das bei der DNA-Replikation beteiligt ist (Watt und Hickson, Biochem. J. 303 (1994), 681-695). Die verringerte Expression der Topoisomerase IIα nach SC-1 induzierter Apoptose zeigt daher eine Zellzyklusarretierung für mindestens einen Teil der Zellen (Figur 14c).

Der Transkriptionsfaktor c-myc ist in verschiedenen apoptotischen Prozessen beteiligt und kann durch Transfektion in Zellen eine Apoptose induzieren (Evan et al., Cell 69 (1992), 119-128). Eine Untersuchung des Expressionsmusters von c-myc nach SC-1 induzierter Apoptose ergab eine erhöhte Expression 15 min nach Induzierung der Apoptose gefolgt von einer Abnahme nach 4 h (Figur 14d).

### 2.14 Wirkung einer Verringerung der extrazellulären und intrazellulären Calciumkonzentration auf die Apoptose

Es wurde untersucht, ob Ca²⁺-Ionen aus dem Zellkulturmedium aufgenommen werden oder aus intrazellulären Ca²⁺-Reservoirs freigesetzt werden. Zur Bestimmung, ob Ca²⁺ aus dem Kulturmedium aufgenommen wird, wurden die Zellen für 24 h in Serum- und Ca²⁺-freiem DMEM-Medium inkubiert. Dann wurde gereinigter SC-1-Antikörper auf eine Endkonzentration von 40 µg/ml zugesetzt und weitere 24 h inkubiert. Die Zellen wurden dann in 3 % Glutaraldehyd fixiert und in einem umgedrehten Lichtmikroskop untersucht. Verglichen mit Kontrollzellen (nicht mit SC-1 induziert) zeigten SC-1 induzierte Zellen morphologische, für eine Apoptose charakteristische Veränderungen, vergleichbar mit Zellen, die mit SC-1 in RPMI-Medium unter Zusatz von 10 % FCS inkubiert worden waren.

Der Einfluß von Ca²⁺ aus intrazellulären Ca²⁺-Reservoirs wurde durch Inkubation von Zellen (kultiviert in serumfreiem DMEM-Medium) für 5 h mit 50µM des zellpermeablen Chelators BAPTA (Alexis Biochemicals, Grünberg, Deutschland) untersucht. Die Zellen wurden für 24 h mit 40 µg/ml gereinigtem SC-1 inkubiert. Es konnten keine erkennbaren Veränderungen in der Zellmorphologie beobachtet werden, was darauf hinweist, daß keine Apoptose induziert wurde. Auch durch ELISA konnte eine durch BAPTA bewirkte Hemmung der Apoptose gefunden werden.

### 2.15 Nachweis von Apoptose in Primärtumoren

Die Verabreichung des Antikörpers SC-1 an Magenkarzinom-Patienten führte zu einer deutlich erkennbaren tumorzellspezifischen Apoptose, wie durch in situ Kernfärbung nachgewiesen wurde (Figur 16). Während in Tumorbiopsien, die vor der Verabreichung von SC-1 entnommen wurden, keine Apoptose (Figur 17a) bzw. das Vorhandensein eines intakten Tumors ohne Regression (Figur 17c) gefunden wurde, zeigte der Primärtumor nach Verabreichung von SC-1 starke Apoptose (Figur 17b) bzw. eine starke Regression (Figur 17d).

## Patentansprüche

1. Isoliertes Glykoprotein umfassend mindestens einen Abschnitt der Aminosäureprimärstruktur von humanem CD55 und eine tumorspezifische Glykostruktur, wobei das Glykoprotein auf einer Magenkazinomzelle vorhanden ist, jedoch nicht auf einer normalen Zelle.

2. Isoliertes Glykoprotein umfassend mindestens einem Abschnitt der Aminosäureprimärstruktur von humanem CD55 und eine tumorspezifische Glykostruktur, wobei das Glykoprotein die Apoptose einer Zelle herbeiführt, wenn es auf der Zelle vorhanden ist und an einen für die Glykostruktur spezifischen Antikörper gebunden ist.

3. Glykoprotein nach Anspruch 1 oder 2,
**dadurch gekennzeichnet,**
**daß** es bei SDS-Polyacrylamid-Gelelektrophorese ein scheinbares Molekulargewicht von 82 kD aufweist.

4. Verfahren zur Gewinnung eines Glykoproteins nach einem der Ansprüche 1 bis 3,
**dadurch gekennzeichnet,**
**daß** man Membranpräparationen aus Zellen der humanen Adenokarzinomzellinie 23132 herstellt und daraus das Glykoprotein durch Größenausschluß-und/oder Anionenaustauschchromatographie gewinnt.

5. Verwendung eines Glykoproteins nach einem der Ansprüche 1 bis 3 in einem Testverfahren, bei dem die Bindefähigkeit einer Substanz an das Glykoprotein bestimmt wird.

6. Verwendung nach Anspruch 5,
**dadurch gekennzeichnet,**
**daß** die Bindefähigkeit an die Glykostruktur bestimmt wird.

7. Verwendung nach Anspruch 5 oder 6,
**dadurch gekennzeichnet,**
**daß** die Fähigkeit der getesteten Substanz zur Apoptose-induzierung, insbesondere bei Tumorzellen bestimmt wird.

8. Verwendung nach einem der Ansprüche 5 bis 7,
**dadurch gekennzeichnet,**
**daß** die Fähigkeit der getesteten Substanz zur Induzierung einer über das Glykoprotein CD55 vermittelten Phosphorylierungskaskade bestimmt wird.

9. Verwendung nach Anspruch 5 bis 8,
**dadurch gekennzeichnet,**
**daß** das Glykoprotein in isolierter Form, als Zellextrakt, insbesondere als Membranpräparation oder in Form vollständiger Zellen, insbesondere der humanen Adenokarzinomzellinie 23132 oder 3051 eingesetzt wird.

10. Verwendung nach einem der Ansprüche 5 bis 9 zur Identifizierung von spezifisch an Tumorzellen bindefähige Substanzen.

11. Verwendung nach Anspruch 10 zur Identifizierung von Mitteln zur Tumordiagnostik oder/und Tumortherapie.

12. Verwendung nach einem der Ansprüche 5 bis 11,
**dadurch gekennzeichnet,**
**daß** pharmakologisch verträgliche Substanzen getestet werden.

13. Verwendung nach Anspruch 12,
**dadurch gekennzeichnet,**
**daß** die getesteten Substanzen aus Peptiden, Peptidmimetika, Antikörpern, Antikörperfragmenten und Antikörperderivaten ausgewählt werden.

14. Glykoprotein nach einem der Ansprüche 1 bis 3 zur Verwendung in einem Verfahren zur Bekämpfung von Tumoren, wobei das Vorhandensein des Glykoproteins auf einer Tumorzelle diese empfindlich für Mittel zur Tumorbekämpfung macht.

15. Glykoprotein nach einem der Ansprüche 1 bis 3 zur Verwendung bei der Diagnose von Tumoren, wobei das Vorhandensein des Glykoproteins in einer Probe darauf hinweist, dass die Probe aus einem Tumor stammt.

16. Glykoprotein nach einem der Ansprüche 1 bis 3, wobei die Bindung der Glykostruktur an einen für die Glykostruktur spezifischen Antikörper keine Spaltung von Poly(ADP-Ribose)-Polymerase (PARP) hervorruft.

17. Glykoprotein nach Anspruch 14, wobei das Mittel zur Tumorbekämpfung eine Zellzyklusarretierung in Tumorzellen induziert.

18. Glykoprotein nach Anspruch 14, wobei das Mittel zur Tumorbekämpfung Apoptose in Tumorzellen induziert.

19. Glykoprotein nach Anspruch 14, 17 oder 18, wobei das Mittel zur Tumorbekämpfung mit einer Markierungs- oder Effektorgruppen konjugiert ist.

20. Glykoprotein nach Anspruch 2 oder 3, wobei die Bindung des Antikörpers an die Glykostruktur zur Spaltung von Cytokeratin 18 führt.

21. Glykoprotein nach Anspruch 2 oder 3, wobei die Bindung des Antikörpers an die Glykostruktur zu einer erhöhten Expression von c-myc führt.

22. Glykoprotein nach Anspruch 2 oder 3, wobei die Bindung des Antikörpers an die Glykostruktur zu einer verringerten Expression von Topoisomerase IIα führt.

23. Glykoprotein nach Anspruch 2 oder 3, wobei die Bindung des Antikörpers an die Glykostruktur zu einer Erhöhung der intrazellularen Ca²⁺-Konzentration führt.

24. Verwendung nach Anspruch 8, wobei die Phosphorylierungskaskade eine Tyrosinphosphorylierungskaskade ist.

## Claims

1. Isolated glycoprotein comprising at least one section of the amino acid primary structure of human CD55 and a tumour-specific glycostructure, wherein the glycoprotein is present on a gastric carcinoma cell but not on a normal cell.

2. Isolated glycoprotein comprising at least one section of the amino acid primary structure of human CD55 and a tumour-specific glycostructure, wherein the glycoprotein induces apoptosis of a cell when it is present on the cell and is bound to an antibody that is specific for the glycostructure.

3. Glycoprotein as claimed in claim 1 or 2,
**characterized in that**
it has an apparent molecular weight of 82 kD in SDS polyacrylamide gel electrophoresis.

4. Process for obtaining a glycoprotein as claimed in one of the claims 1 to 3,
**characterized in that**
membrane preparations are produced from cells of the human adenomacarcinoma cell line 23132 and the glycoprotein is obtained therefrom by size-exclusion and/or anion exchange chromatography.

5. Use of a glycoprotein as claimed in one of the claims 1 to 3 in a test procedure in which the ability of a substance to bind to the glycoprotein is determined.

6. Use as claimed in claim 5,
**characterized in that**
the ability to bind to the glycostructure is determined.

7. Use as claimed in claim 5 or 6,
**characterized in that**
the ability of the tested substance to induce apoptosis especially in tumour cells is determined.

8. Use as claimed in one of the claims 5 to 7,
**characterized in that**
the ability of the tested substance to induce a phosphorylation cascade mediated by the glycoprotein CD55 is determined.

9. Use as claimed in claims 5 to 8,
**characterized in that**
the glycoprotein is used in an isolated form, as a cell extract, especially as a membrane preparation or in the form of a complete cells especially of the human adenocarcinoma cell line 23132 or 3051.

10. Use as claimed in one of the claims 5 to 9 to identify substances that are able to bind specifically to tumour cells.

11. Use as claimed in claim 10 to identify agents for tumour diagnosis and/or tumour therapy.

12. Use as claimed in one of the claims 5 to 11,
**characterized in that**
pharmacologically compatible substances are tested.

13. Use as claimed in claim 12,
**characterized in that**
the tested substances are selected from peptides, peptide mimetic agents, antibodies, antibody fragments and antibody derivatives.

14. Glycoprotein as claimed in one of the claims 1 to 3 for use in a method for treating tumours in which the presence of the glycoprotein on a tumour cell makes it sensitive to agents for treating tumours.

15. Glycoprotein as claimed in one of the claims 1 to 3 for use in the diagnosis of tumours in which the presence of the glycoprotein in a sample indicates that the sample is derived from a tumour.

16. Glycoprotein as claimed in one of the claims 1 to 3 wherein the binding of the glycostructure to an antibody that is specific for the glycostructure does not result in a cleavage of poly(ADP-ribose) polymerase (PART).

17. Glycoprotein as claimed in claim 14, wherein the anti-tumour agent induces a cell-cycle arrest in tumour cells.

18. Glycoprotein as claimed in claim 14, wherein the anti-tumour agent induces apoptosis in tumour cells.

19. Glycoprotein as claimed in claim 14,17 or 18, wherein the anti-tumour agent is conjugated with a labelling or effector group.

20. Glycoprotein as claimed in claim 2 or 3, wherein the binding of the antibody to the glycostructure results in the cleavage of cytokeratin 18.

21. Glycoprotein as claimed in claim 2 or 3, wherein the binding of the antibody to the glycostructure results in an increased expression of c-myc.

22. Glycoprotein as claimed in claim 2 or 3, wherein the binding of the antibody to the glycostructure results in a reduced expression of topoisomerase IIα.

23. Glycoprotein as claimed in claim 2 or 3, wherein the binding of the antibody to the glycostructure results in an increase in the intracellular Ca²⁺ concentration.

24. Use as claimed in claim 8, wherein the phosphorylation cascade is a tyrosine phosphorylation cascade.

## Revendications

1. Glycoprotéine isolée comprenant au moins un segment de la structure primaire d'acides aminés du CD55 humain et une glycostructure tumeur-spécifique, la glycoprotéine étant présente sur une cellule de carcinome gastrique, mais pas sur une cellule normale.

2. Glycoprotéine isolée comprenant au moins un segment de la structure primaire d'acides aminés du CD55 humain et une glycostructure tumeur-spécifique, la glycoprotéine entraînant l'apoptose d'une cellule, lorsqu'elle est présente sur la cellule et liée à un anticorps spécifique de la glycostructure.

3. Glycoprotéine selon la revendication 1 ou 2,
**caractérisée en ce que**,
elle présente, lors d'une l'électrophorèse sur gel SDS polyacrylamide, une masse moléculaire apparente de 82 kD.

4. Procédé permettant l'obtention d'une glycoprotéine selon l'une quelconque des revendications 1 à 3,
**caractérisé en ce que**,
on confectionne des préparations membranaires à partir de cellules de la. lignée de cellules d'adénocarcinome humain 23132 et on obtient à partir de là la glycoprotéine par chromatographie d'exclusion stérique et/ou d'échange d'anions.

5. Utilisation d'une glycoprotéine selon l'une quelconque des revendications 1 à 3 dans une méthode de test dans laquelle on détermine la capacité d'une substance à se lier à la glycoprotéine.

6. Utilisation selon la revendication 5,
**caractérisée en ce que**,
on détermine la capacité à se lier à la glycostructure.

7. Utilisation selon la revendication 5 ou 6,
**caractérisée en ce que**
on détermine la capacité de la substance testée à induire l'apoptose, en particulier pour des cellules tumorales.

8. Utilisation selon l'une quelconque des revendications 5 à 7,
**caractérisée en ce que**
on détermine la capacité de la substance testée à induire une cascade de phosphorylation médiée par la glycoprotéine CD55.

9. Utilisation selon les revendications 5 à 8,
**caractérisée en ce que**
la glycoprotéine est utilisée sous forme isolée, en tant qu'extrait cellulaire, en particulier en tant que préparation membranaire ou sous forme de cellules entières, en particulier de la lignée de cellules d'adénocarcinome humain 23132 ou 3051.

10. Utilisation selon l'une quelconque des revendications 5 à 9 pour l'identification de substances pouvant se lier à des cellules tumorales de façon spécifique.

11. Utilisation selon la revendication 10 pour l'identification d'agents pour le diagnostic de tumeurs et/ou le traitement de tumeurs.

12. Utilisation selon l'une quelconque des revendications 5 à 11,
**caractérisée en ce que**
on teste des substances pharmacologiquement compatibles.

13. Utilisation selon la revendication 12,
**caractérisée en ce que**,
les substances testées sont choisies parmi les peptides, les mimétiques de peptides, les anticorps, les fragments d'anticorps et des dérivés d'anticorps.

14. Glycoprotéine selon l'une quelconque des revendications 1 à 3 destinée à être utilisée dans un procédé de traitement contre les tumeurs, la présence de la glycoprotéine sur une cellule tumorale rendant celle-ci sensible aux agents de traitement contre les tumeurs.

15. Glycoprotéine selon l'une quelconque des revendications 1 à 3 destinée à être utilisée en diagnostic de tumeurs, la présence de la glycoprotéine dans un échantillon indiquant que l'échantillon provient d'une tumeur.

16. Glycoprotéine selon l'une quelconque des revendications 1 à 3, la liaison de la glycostructure à un anticorps spécifique de la glycostructure ne provoquant pas de séparation de la poly(ADP-ribose)-polymérase (PARP).

17. Glycoprotéine selon la revendication 14, l'agent de traitement contre les tumeurs induisant un arrêt du cycle cellulaire dans les cellules tumorales.

18. Glycoprotéine selon la revendication 14, l'agent de traitement contre les tumeurs induisant une apoptose dans les cellules tumorales.

19. Glycoprotéine selon la revendication 14, 17 ou 18, l'agent de traitement contre les tumeurs étant conjugué avec un groupement marqueur ou effectueur.

20. Glycoprotéine selon la revendication 2 ou 3, la liaison de l'anticorps à la glycostructure entraînant la séparation de la cytokératine 18.

21. Glycoprotéine selon la revendication 2 ou 3, la liaison de l'anticorps à la glycostructure entraînant une expression accrue de c-myc.

22. Glycoprotéine selon la revendication 2 ou 3, la liaison de l'anticorps à la glycostructure entraînant une expression réduite de la topoisomérase IIα.

23. Glycoprotéine selon la revendication 2 ou 3, la liaison de l'anticorps à la glycostructure entraînant une augmentation de la concentration de Ca²⁺ intracellulaire.

24. Utilisation selon la revendication 8, la cascade de phosphorylation étant une cascade de phosphorylation de la tyrosine.
